# EUROPEAN PATENT APPLICATION

(11) **EP 3 292 865 A1**
(43) Date of publication of application: **14.03.2018**
(21) Application number: 16789007.8
(22) Date of filing: 11.04.2016
(51) Int. Cl.: A61K 31/12, A61P 35/00, A61P 7/06

(54) **USE OF CURCUMIN AND PHARMACEUTICALLY ACCEPTABLE SALT THEREOF**

(30) Priority: 06.05.2015 CN 201510227963
(71) Applicant: Targetpharma Laboratories (Changzhou) Co., Ltd, Changzhou 213164 (CN); Changzhou High-Tech Research Institute of Nanjing University, Changzhou, Jiangsu 213164 (CN)
(72) Inventor: HUA, Zichun, Changzhou Jiangsu 213164 (CN); FU, Zhongping, Changzhou Jiangsu 213164 (CN); CHEN, Xiao, Changzhou Jiangsu 213164 (CN); GUAN, Shengwen, Changzhou Jiangsu 213164 (CN)
(74) Representative: Kilger, Ute
(86) International application number: PCT/CN2016/000188
(87) International publication number: WO 2016/177013

(57) **Abstract**

Disclosed is the use of a curcumin and a pharmaceutically acceptable salt thereof in the preparation of drugs for antitumor associated syndrome. Curcumin can improve anemia while inhibiting tumors.

## Description

### Technical Field

The invention relates to an antineoplastic agent, in particular to the use of curcumin and pharmaceutically acceptable salt thereof in the preparation of drugs for antitumor associated syndrome.

### Background of the Invention

Tumor associated syndrome seriously affects the quality of patients' life, which is an important cause of death among cancer patients. Tumor associated syndrome includes hematopoietic dysfunction, endocrine disorders, ascites, liver injuries, spleen injuries, renal function impairment, gastrointestinal dysfunction, muscle atrophy and so on. For example, anemia is a common complication in patients with malignant tumors, and it is reported that more than 50% of cancer patients suffer from anemia. The severity of anemia is associated with many factors, including disease progression, the type of chemotherapeutic agent used, age increasing and occurrence of latent infections and so on. The incidence rates of anemia differ in terms of different tumors: 81% of gynecologic tumors, 77% of lung cancer, 73% of lymphoma and myeloma, 61% of gastrointestinal tumors, 62% of breast cancer, and 51% of genitourinary tumors. Course of Chemotherapy is closely related to the incident rate of anemia and severity of anemia. Coiffier et al. carried out a survey enrolled more than 1000 cancer patients showing that there was an increase in the incidence rate of anemia after 1 month of chemotherapy, and showing that more than 50% of patients suffered moderate anemia (hemoglobin 8 ∼ 10.0g /dl) or severe (hemoglobin <8.0g /dl) anemia after 10 to 12 months of chemotherapy. Effective control and treatment of anemia and other tumor associated syndrome are of great significance in the comprehensive treatment of tumors.

### Summary of the Invention

Technical problem needs to be solved in this invention is to provide a new use of curcumin, which is particularly related to the use of curcumin and pharmaceutically acceptable salts thereof in the preparation of drugs for antitumor associated syndrome.

In order to solve the above-mentioned technical problem, technical solution adopted in the present invention is directed to the use of curcumin and pharmaceutically acceptable salt thereof in the preparation of drugs for antitumor associated syndrome.

Preferably, the tumor discussed herein is fibrosarcoma or NSCLC (Non Small Cell Lung Cancer).

Preferably, the drugs for antitumor associated syndrome discussed herein are formulated in many forms, including liquid preparations, granules, tablets, powders, capsules, sustained-release preparations, orally disintegrating preparations, or injections.

Preferably, the pharmaceutically acceptable salt of curcumin discussed herein is the salt formed by curcumin and inorganic acid.

Preferably, the inorganic acid discussed herein comprises at least one of hydrochloric acid, hydrobromic acid, sulfuric acid, nitric acid and phosphoric acid.

Preferably, the pharmaceutically acceptable salt of curcumin is the salt formed by curcumin and organic acid.

Preferably, the organic acid discussed herein comprises at least one of formic acid, acetic acid, propionic acid, oxalic acid, malonic acid, succinic acid, fumaric acid, maleic acid, lactic acid, malic acid, tartaric acid, citric acid, picric acid, methanesulfonic acid and methylbenzenesulfonic acid.

Preferably, the pharmaceutically acceptable salt of curcumin discussed herein is the salt formed by curcumin and acid of acidic amino acid.

Preferably, the acidic amino acid discussed herein is aspartic acid or glutamic acid.

The invention is also directed to the use of plants with curcumin as the main ingredient in the preparation of drugs or food for antitumor associated syndrome.

Beneficial effects of the invention are summarized as follows:
1. Conventional antitumor chemotherapeutic agents are not able to avoid or mitigate tumor associated syndrome. Worse still, they will strongly increase the incidence rate of tumor associated syndrome (such as anemia) and its severity. Instead, curcumin can both inhibit tumor growth and remarkably inhibit tumor associated syndrome, providing a new way of preparing the drug for antitumor associated syndrome.
2. The pharmaceutically acceptable salt of curcumin can maintain the same effect as curcumin and provide more preservation ways of curcumin, which makes curcumin to have a more widely application.
3. Curcumin and pharmaceutically acceptable salt thereof, as the main ingredient of drugs for antitumor associated syndrome, take effect in the treatment of tumor while remarkably inhibiting the occurrence of antitumor associated syndrome, showing a better medicinal effect. Thus, it is possible for mass production and further promotion and use in the future.
4. Curcumin is a phenolic pigment extracted from the rhizome of turmeric, which has been used as food coloring in products, such as sausage product, canned food, and sauced marinated product. Curcumin is certain raw material for food. Therefore, plants with curcumin being the main active ingredient can be used in preparing foods or drugs for antitumor associated syndrome.

### Brief Description of the Drawings

Figure 1 is the experimental result of curcumin used in the treatment of tumors in an animal model.
Figure 2 is the experimental result of curcumin treatment's influence on mouse tumor weight and liver and spleen coefficients.
Figure 3 is the experimental result of anemia symptom analysis after curcumin treatment.

### Detailed Description of the invention

### Example 1: Experiment of antitumor animal model

At present, conventional antitumor drugs are accompanied with toxic side effects while taking effect. In this experiment, we evaluated the antitumor effect of curcumin, and examined the toxicity of the drug by observing changes in animals' body weights after treatment. Given that the chemotherapy drug treatment and course of chemotherapy are closely related to the incident rates of antitumor associated syndrome and its severity, positive control in this experiment used the most advanced antitumor antibodies in the world as a control. T241-VEGF is a tumor model with rapid tumor growth and obvious symptom of tumor associated syndrome.

We feed C57BL/6 female mice (SPF grade, 6-8 weeks old, manufactured by Shanghai Silaike Experimental Animal Co., Ltd.) for 2-3 days. The day before subcutaneous inoculation, body hair in back and neck was shaved with a shaving device for the preparation of subcutaneous inoculation of tumor cells. The tumor cells T241-VEGF were collected and washed twice with PBS. After resuspended with PBS and counted, the cells were diluted to 1×10⁷ cells /ml with PBS and placed on ice ready for inoculation. Mice were anesthetized with 7% chloral hydrate, 0.1 ml/each, intraperitoneal injection. After the mice were completely anesthetized, the mice were subcutaneously inoculated with tumor cells T241-VEGF, 1×10⁶/each, with 1ml medical disposable syringes in the back and neck. Inoculated mice were randomly allocated (8 in each group), weighed, grouped and administered for treatment. In this experiment, curcumin (manufactured by Sigma, cat.no.C1386-10G) was dissolved to 1 mol/L with DMSO and diluted to 2.5 mM, 5 mM and 10 mM with 20% Tween-80 (mixed with PBS) before administration. Detailed information and administration plan is as follows: Control: 20 % Tween-80, 0.1ml/each; The low-dose of curcumin group (Curcumin-2.5mM): 2.5 mM curcumin, 0.1 ml/each, continuous administration for 14 days; The medium-dose of curcumin group (Curcumin-5mM): 5 mM curcumin, 0.1 ml/each, continuous administration for 14 days; The high-dose of curcumin group (Curcumin-lOmM): 10mM Curcumin, 0.1 ml/each, continuous administration for 14 days; Positive control group (PC: anti-VEGF humanized monoclonal antibody, produced by Jiangsu Simcere Pharmaceutical Co., Ltd.): 2mg/ml, 0.1ml/each, twice a week , administration for 4 times. Mice were weighed up every other day. Measuring the tumor volume with vernier caliper every other day after tumor grew in the body.

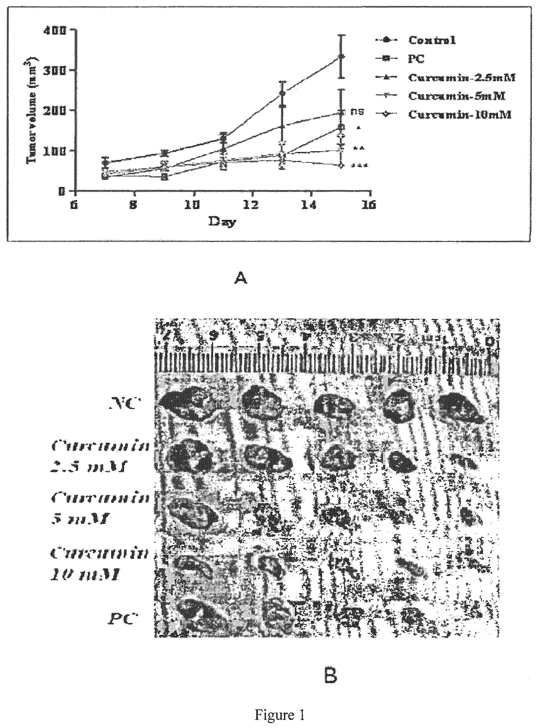

The results, shown in Figure 1-A, indicate that the low, medium and high dose of curcumin can inhibit the growth of T241-VEGF tumors. Compared with the control group, the final tumor volume of groups treated at doses of 5mM and 10mM are significantly different (p <0.01 or p <0.001), and mice tumor volume of 2.5mM group has been inhibited to a certain extent. The tumor weight results corresponding to the tumor volume results are shown in Figure 1-B, showing that the tumor weights of the 5 mM and 10 mM dose groups are significantly different from those in the control group, and indicating that both the 5 mM and 10 mM doses of curcumin significantly inhibit T241-VEGF Tumor growth. The body weight in each groups with the low, medium and high dose of curcumin is not reduced, and is obviously better than the positive control drug, showing that the toxicity is even better than the current international most advanced anti-tumor antibody drugs. The body weight changes in the groups with the low, medium and high dose of curcumnin are consistent with those of animals feed in a normal way in the same period. However, the body weights of animals in the positive control group are significantly lower than those of normal animals and animals with the low, medium and high doses of curcumin.

### Example 2: Experiment on tumor weight and liver and spleen coefficients of mice in each group

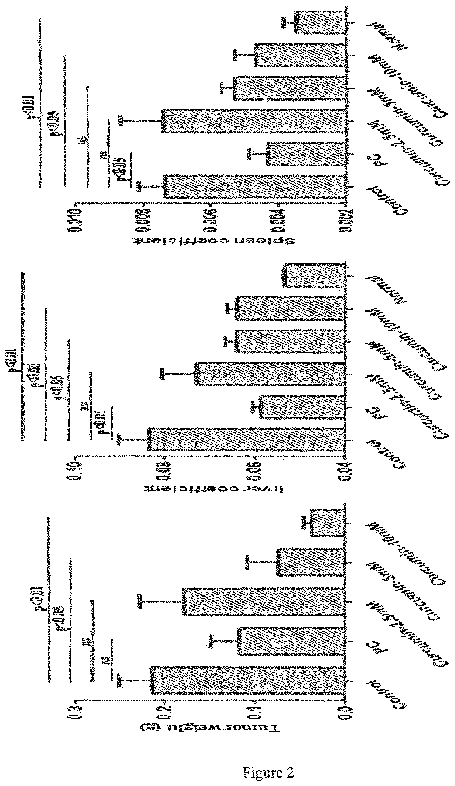

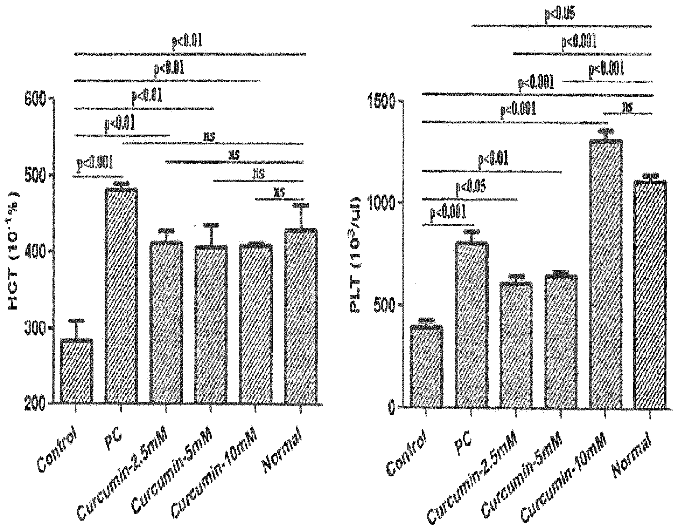

In order to further verify the antitumor effect of each dose of curcumin and its effect on liver and spleen, we handled and dissected the animals in each group the day after administration, got tumors, livers, spleens and kidneys, then weighed up all the tissues and stored them respectively. As shown in Fig. 2, curcumin has an obvious improved effect on liver enlargement and splenomegaly caused by T241-VEGF tumor. The results showed that the liver coefficient and spleen coefficient of the mice in the tumor control group were significantly different from those in the normal mice (p <0.01). 5mM and 10mM curcumin could significantly improve the liver enlargement (p <0.05), while 10mM curcumin can significantly improve the spleen enlargement (p <0.05).

### Example 3: Experiment on analysis of anemia symptom of mice in each group

Handled the animals in each group the day after administration, weighed up, then collected blood (stored in 1.5 ml of EP tubes with 20ul of 0.5M EDTA) after enucleation of the eyeballs and performed blood analysis. Blood samples were taken to evaluate the effect of curcumin on tumor associated syndrome- red blood cell (RBC), hemoglobin (HGB), hematocrit (HCT) and platelet count (PLT) in mice.

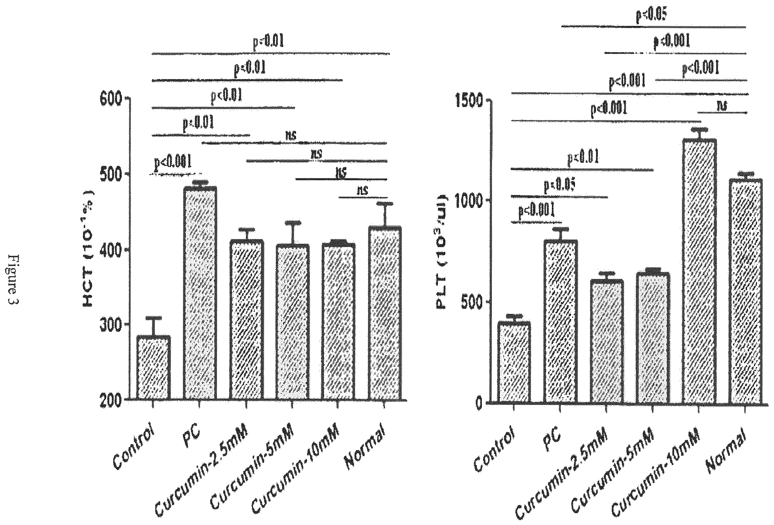

As for tumor associated syndrome, such as mouse anemia symptoms, induced by the T241-VEGF tumor animal model, we performed blood analysis on the blood of mice in each group. The results were shown in Figure 3: Compared with the normal mice, red blood cell (RBC), hemoglobin (HGB), hematocrit (HCT) and platelet count (PLT) of mice in the Control group were significantly reduced, indicating that T241-VEGF tumor model can produce tumor associated syndrome, that is to say, anemia symptom of tumor-bearing mouse. Positive monoclonal antibody drug can significantly improve the anemia symptoms of tumor-bearing mice, but the positive monoclonal antibody drug would lead to increase in red blood cell (RBC), hemoglobin (HGB), hematocrit (HCT). As for mice treated at the low, medium and high dose of curcumin, the anemia symptoms were improved remarkably, and the improvement of the anemia symptoms was significantly different from those of control group. While compared with normal mice, there was no statistical differences in terms of red blood cell (RBC), hemoglobin (HGB), hematocrit (HCT), and when administered at a dose of 10mM, there was no statistical differences in terms of platelet, indicating that curcumin can remarkably improve the anemia symptoms induced by tumor.

In the meantime, the result obtained from an experiment based on the tumor model of NSCLS A549 was almost the same with that of the above-mentioned experiment.

This result fully shows: in light of currently known six mechanisms of action of curcumin for the treatment of antitumor, namely, inducing apoptosis, inducing cell cycle arrest, blocking of tumor cell growth signal transduction, inhibiting tumor angiogenesis, inhibiting the expression of adhesion molecules, increasing sensitivity of chemotherapeutic agents to cancer cells (International Journal of Oncology 2013, v40(11): 823-826), curcumin is expected to be similar to the chemotherapeutic agents (inducing apoptosis, inducing cell cycle arrest, blocking of tumor cell growth signal transduction, increasing sensitivity of chemotherapeutic agents to cancer cells, all of which are common features of action among many small molecule chemotherapeutic agents), having influence on increasing the incidence rate of tumor associated syndrome and the severity thereof. However, the present invention has surprisingly found that curcumin can remarkably inhibit tumor growth and tumor associated syndrome, while the body weight of tumor- bearing animal grows in the way that normal animal does without any toxic side effects.

From the Example 1-3 based on pharmaceutically acceptable salt formed by curcumin and inorganic acid, or formed by curcumin and organic acid, or formed by curcumin and acid of acidic amino acid (Examples of inorganic acid include hydrochloric acid, hydrobromic acid, sulfuric acid, nitric acid, phosphoric acid and the like. Examples of organic acid include formic acid, acetic acid, propionic acid, oxalic acid, malonic acid, succinic acid, fumaric acid, maleic acid, lactic acid, malic acid, tartaric acid, citric acid, picric acid, methanesulfonic acid, methylbenzenesulfonic acid, and the like. Examples of acid of acidic amino acid include aspartic acid, glutamic acid and the like.), it is found that pharmaceutically acceptable salt of curcumin has the same effect of curcumin. In further application, according to the actual need, drugs for antitumor associated syndrome comprising curcumin and pharmaceutically acceptable salt thereof can be formulated in many forms, namely, liquid preparations, granules, tablets powders, capsules, sustained-release preparations, orally disintegrating preparations, or injections.

Result from the Examples 1-3 based on animal food made from raw materials of ginger, mustard and curry etc. which are enriched in curcumin, it suggests that food rich in curcumin has similar effect of curcumin. In further application, according to the actual needs, food for antitumor associated syndrome comprising curcumin can be in many forms of liquid preparations, granules, tablets, powder, capsule, sustained-release preparations, and orally disintegrating preparations.

In conclusion, curcumin can both inhibit tumor growth and remarkably inhibit tumor associated syndrome, providing a new way of developing new drugs for antitumor associated syndrome.

## Claims

1. Use of curcumin and pharmaceutically acceptable salt thereof in the preparation of drugs for antitumor associated syndrome.

2. The use of claim 1, wherein the tumor is fibrosarcoma or NSCLC.

3. The use of claim 1, wherein the drugs for antitumor associated syndrome are formulated in many forms, including liquid preparations, granules, tablets, powders, capsules, sustained-release preparations, orally disintegrating preparations, or injections.

4. The use of any one of claims 1-3, wherein the pharmaceutically acceptable salt of curcumin is the salt formed by curcumin and inorganic acid.

5. The use of claim 4, wherein the inorganic acid comprises at least one of hydrochloric acid, hydrobromic acid, sulfuric acid, nitric acid and phosphoric acid.

6. The use of any one of claims 1-3, wherein the pharmaceutically acceptable salt of curcumin is the salt formed by curcumin and organic acid.

7. The use of claim 6, wherein the organic acid comprises at least one of the formic acid, acetic acid, propionic acid, oxalic acid, malonic acid, succinic acid, fumaric acid, maleic acid, lactic acid, malic acid, tartaric acid, citric acid, picric acid, methanesulfonic acid and methylbenzenesulfonic acid.

8. The use of any one of claims 1-3, wherein the pharmaceutically acceptable salt of curcumin is the salt formed by curcumin and acid of acidic amino acid.

9. The use of claim 8 wherein the acidic amino acid is aspartic acid or glutamic acid.

10. Use of plants with curcumin as the main ingredient in the preparation of drugs or food for antitumor associated syndrome.
